# EUROPEAN PATENT SPECIFICATION

(11) **EP 1 368 015 B1**
(45) Date of publication and mention of the grant of the patent: **09.08.2006**
(21) Application number: 02711877.7
(22) Date of filing: 21.02.2002
(51) Int. Cl.: A61K 31/133, A61K 31/137, A61K 31/201, A61P 37/06

(54) **USE OF ACCELERATED LYMPHOCYTE HOMING AGENTS FOR THE MANUFACTURE OF A MEDICAMENT FOR THE TREATMENT OF DELAYED GRAFT FUNCTION**
VERWENDUNG VON BESCHLEUNIGTEN LYMPHOZYTEN ZIELANSTEUERNDEN WIRKSTOFFEN ZUR HERSTELLUNG EINES MEDIKAMENTS FÜR DIE BEHANDLUNG DER VERZÖGERTERN TRANSPLANTAT-FUNKTION
UTILISATION D'AGENTS DE HOMING LYMPHOCYTAIRE ACCELERE POUR LA PRODUCTION D'UN MEDICAMENT DESTINE AU TRAITEMENT DE LA REPRISE FONCTIONNELLE RETARDEE DU GREFFON

(30) Priority: 22.02.2001 GB 0104438; 28.02.2001 GB 0105000
(43) Date of publication of application: 10.12.2003
(73) Proprietor: Novartis AG, 4056 Basel (CH); Novartis Pharma GmbH, 1230 Vienna (AT)
(72) Inventor: BIGAUD, Marc, F-682800 Sundhoffen (FR); BRINKMANN, Volker, 79102 Freiburg (DE); SABLINSKI, Tomasz, CH-4057 Basel (CH)
(74) Representative: de Weerd, Petrus G.W.
(86) International application number: PCT/EP2002/001860
(87) International publication number: WO 2002/067915

(56) References cited:
- EP-A- 0 627 406
- US-A- 3 928 572
- US-A- 6 004 565
- LUO ZHI-JUAN ET AL: "Analysis of the mode of action of a novel immunosuppressant FTY720 in mice." IMMUNOPHARMACOLOGY, vol. 41, no. 3, April 1999 (1999-04), pages 199-207, XP002198037 ISSN: 0162-3109

## Description

The present invention relates to a new use for an accelerated lymphocyte homing ("ALH") agent e.g. a 2-amino-1,3-propanediol derivative.

The ALH agents of the invention are compounds which sequester lymphocytes from peripheral tissues to secondary lymphatic organs. Suitable ALH agents include e.g. analogs from myriocin or ISP-1, a natural metabolite of the ascomycetes *Isaria sinclainï.* Suitable ALH are e.g. 2-aminopropane-1,3-diol compounds of formula I: 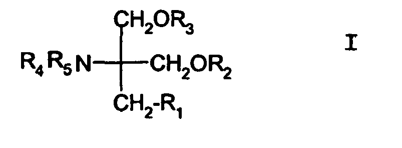 wherein
R₁ is an optionally substituted straight- or branched carbon chain having 12 to 22 carbon atoms which may be optionally interrupted by an optionally substituted phenylene, and
each of R₂, R₃, R₄ and R₅, independently, is H or lower alkyl,

in free form or in pharmaceutically acceptable salt form.

When the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is a straight or branched, preferably straight, chain alkyl having 13 to 20 carbon atoms, optionally substituted by nitro, halogen, amino, hydroxy or carboxy, and, more preferably those wherein R₁ is phenylalkyl substituted by a straight or branched C₈₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

When the compounds of formula I have one or more asymmetric centers in the molecule, the present invention is to be understood as embracing the various optical isomers, as well as racemates, diastereoisomers and mixtures thereof.

Examples of the pharmaceutically acceptable salts of the compounds of formula I include salts with inorganic acids, such as hydrochloride, hydrobromide and sulfate, salts with organic acids, such as acetate, fumarate, maleate, benzoate, citrate, malate, methanesulfonate and benzenesulfonate salts, and when a carboxy group is present, salts with metals such as sodium, potassium, calcium and aluminium, salts with amines, such as triethylamine and salts with dibasic amino acids, such as lysine. Compounds of formula I and salts of the present invention encompass hydrate and solvate forms.

A preferred compound of formula I is 2-amino-2-tetradecyl-1,3-propanediol. A particularly preferred ALH agent for use in the invention is FTY720, i.e. 2-amino-2-[2-(4-octylphenyl) ethyl]propane-1,3-diol, in free form or in pharmaceutically acceptable salt form (hereinafter referred to as Compound A), e.g. the hydrochloride salt , as shown:

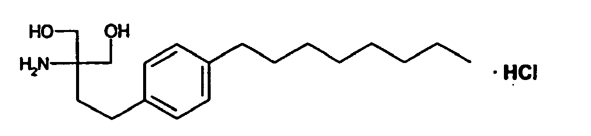

Compounds of formula I have, on the basis of observed activity, e.g. as described in EP-A1-627,406 been found to be useful e.g. as immunosuppressants, e.g. in the treatment of acute allograft rejection or autoimmune disorders.

In accordance with the present invention, it has now surprisingly been found that a ALH agent, e.g. Compound A in free form or in pharmaceutically acceptable salt form, reduces delayed graft function.

Following renal transplantation, approximately 25% of patients exhibit a delay in the onset of renal function in most instances requiring e,g, intervention with hemodialysis; this condition is referred as delayed graft function (DGF). This incidence has remained stable since 1990, despite the introduction of a number of new immunosuppressive drugs in the last decade. The major risk factors that are associated with an increased incidence of DGF are prolonged cold ischemia time and procurement of kidneys from older donors or cadaveric kidneys.

DGF is a dramatic consequence of prolonged cold preservation of an organ prior to its transplantation into a recipient. It is characterized by an impaired functional recovery of the grafts upon reperfusion and often results in an increased need of post-operative care. In case of heart transplantation, DGF may result in death, temporary heart failure and/or in a need for chronotropic (external pacing) or inotropic (pharmacologic agents) supports. In case of kidney transplantation, DGF may result in temporary kidney failure requiring pharmacological treatments and/or additional dialysis. The severity of DGF is proportional to the duration of cold preservation. Any treatment reducing DGF will not only improve the functional recovery of all grafts subjected to cold preservation but also will reduce the number of grafts that cannot be used because of too long preservation time (so called non-optimal grafts). Ultimately, this may increase the number of grafts available.

Immunosuppressive management of patients with DGF still remains a major unmet medical need in transplantation.

In accordance with the particular findings of the present invention, there is provided:
1.1. A method of reducing DGF in a recipient of organ or tissue transplant comprising administering to said recipient a therapeutically effective amount of an ALH agent, e.g. Compound A in free form or in pharmaceutically acceptable salt form;
1.2 A method of improving functional recovery of a transplanted organ or tissue in a recipient of organ or tissue transplant comprising administering to said recipient a therapeutically effective amount of a ALH agent, e.g. Compound A in free form or in pharmaceutically acceptable salt form.

Organ or tissue transplants include e.g. heart, lung, combined heart-lung, liver, kidney, spleen, small bowell, pancreatic (complete or partial, e.g. Langerhans islets) grafts, bone marrow or stem cells.
1.3 A method of improving glomerular filtration rate of a transplanted kidney in a recipient of kidney transplant comprising administering to said recipient a therapeutically effective amount of a ALH agent, e.g. Compound A in free form or in pharmaceutically acceptable salt form.
1.4 A method as defined above, comprising co-administration of a therapeutically effective amount of an ALH agent, e.g. Compound A in free form or in pharmaceutically acceptable salt form, and a second drug substance, said second drug substance being an immunosuppressant or immunomodulatory drug.
1.5 A method as defined above, comprising co-administration of a therapeutically effective amount of an ALH agent, e.g. Compound A in free form or in pharmaceutically acceptable salt form, and a second drug substance, said second drug substance being an immunosuppressant or immunomodulatory drug other than a calcineurin inhibitor.

Suitable second drug substances may include e.g. a calcineurin inhibitor, e.g. cyclosporin A or FK-506; a macrocyclic lactone which exhibits immunosuppressant properties, e.g. rapamycin or a derivative thereof, e.g. 40-O-(2-hydroxyethyl)-rapamycin or 40-[3-hydroxy-2-(hydroxymethyl)-2-methylpropanoate]-rapamycin; corticosteroids; cyclophosphamide; azathioprine; methotrexate; brequinar; leflunomide; mizoribine; mycophenolic acid; mycophenolate mofetil; 15-deoxyspergualine or a derivative thereof; immuno-suppressive monoclonal antibodies, e.g., monoclonal antibodies to leukocyte receptors, e.g., to MHC, CD2, CD3, CD4, CD7, CD11a/CD18, CD25, CD28, B7, CD40, CD45, CD58, CD137, ICOS, CD150, OX40, 4-1BB or to their ligands; or other immunomodulatory compounds, e.g.

CTLA4-1g or an analog, homolog or derivative thereof, e.g. LEA29Y.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.
2. An ALH agent, e.g. Compound A in free form or in pharmaceutically acceptable salt form, for use in any method as defined under 1.1 to 1.5 above; or
3. An ALH agent, e.g. Compound A in free form or in pharmaceutically acceptable salt form, for use in the preparation of a pharmaceutical composition for use in any method as defined under 1.1 to 1.5 above; or
4. A pharmaceutical composition for use in any method as defined under 1.1 to 1.5 above comprising an ALH agent, e.g. Compound A in free form or in pharmaceutically acceptable salt form, together with one or more pharmaceutically acceptable diluents or carriers therefor.
5. A pharmaceutical combination comprising:
   a) a first agent which is an ALH agent, e.g. Compound A in free form or in pharmaceutically acceptable salt form, and
   b) a co-agent which is an immunosuppressant or immunomodulatory drug, e.g. as disclosed above,
   e.g. for use in any method as defined under 1.1 to 1.5 above. Preferably, the co-agent b) is an immunosuppressant or immunomodulatory drug other than a calcineurin inhibitor, e.g. as disclosed above.

The term "pharmaceutical combination" as used herein means a product that results from the mixing or combining of more than one active ingredient and includes both fixed and non-fixed combinations of the active ingredients. The term "fixed combination" means that the active ingredients, e.g. Compound A and a co-agent, are both administered to a patient simultaneously in the form of a single entity or dosage. The term "non-fixed combination" means that the active ingredients, e.g. Compound A and a co-agent, are both administered to a patient as separate entities either simultaneously, concurrently or sequentially with no specific time limits, wherein such administration provides therapeutically effective levels of the agents in the body of the patient. By "co-agent" is meant one or more compounds as disclosed above.

Preferably the ALH agent e.g. Compound A in free form or in pharmaceutically acceptable salt form, is co-administered with an immunosuppressant or immunomodulatory drug, e.g. a calcineurin inhibitor, e.g. cyclosporin A, or a macrocyclic lactone which exhibits immunosuppressant properties, e.g. rapamycin or a derivative thereof.

A preferred combination according to the invention comprises Compound A in free form or in pharmaceutically acceptable salt form, e.g. hydrochloride salt, and, as co-agent b), either rapamycin or a derivative thereof, e.g. 40-O-(2-hydroxyethyl)-rapamycin, optionally together with a corticosteroid.

Utility of the ALH agents, e.g. Compound A in free form or in pharmaceutically acceptable salt form, e.g. in the reduction of DGF or improvement of the functional graft recovery, as hereinabove specified, may be demonstrated in animal test methods as well as in dinic, for example in accordance with the methods hereinafter described.

### A. In vivo

Kidneys from DA rats are preserved 40h at 4°C prior to transplantation and then transplanted as life supporting allografts in Lewis rats. DGF is assessed during 1 week post transplantation by monitoring glomerular filtration rate (GFR). Recipients are treated with the ALH agent to be tested, either alone, or combined with a macrocyclic lactone, e.g. 40-O-(2-hydroxyethyl)-rapamycin, or a calcineurin inhibitor, e.g. cyclosporin A in a microemulsion. In the control groups, cyclosporin A is given alone at 5 mg/kg/day, a dose which increases graft survival to at least 80 days. In this assay, the ALH agent whether administered alone or in combination with a calcineurin inhibitor or a macrocyclic lactone improves DGF over the controls.

More particularly, in control animals GFR is reduced by about 65% at 1 week post transplantation, whereas in recipients treated with 0.3 mg/kg/d Compound A hydrochloride + 0.625 mg/kg/d 40-O-(2-hydroxyethyl)-rapamycin the reduction of GFR is only 35% at 1 week post transplantation.

### B. Clinical Trial

30 to 60 de novo adult renal transplant recipients at increased risk of DGF are included in a one year open-label study. Baseline assessment occurs within 24 hours pre-transplantation. A core renal allograft biopsy is performed prior to implantation and revascularization on Day 0, to serve as a Baseline comparison for evaluation of subsequent biopsies should they be required. Each patient must meet inclusion criteria and none of the exclusion criteria, at Baseline (pre-transplantation) and immediately prior to enrollment in order to participate in this study.

Patients receive a first dose of ALH agent, e.g. Compound A in free form or a pharmaceutically acceptable salt thereof, e.g. orally, at least 2 hours prior to the renal allograft revascularization (Day 0). Maintenance immunosuppression with the ALH agent commences on the morning following transplantation (D 1) The once daily dose is then adjusted for each patient as required.

Patients are treated with their first dose of 40-O-(2-hydroxyethyl)-rapamycin at least 2 hours prior to renal allograft revascularization (Day 0), concurrently with the ALH agent dose. All patients then receive daily an ALH agent dose, e.g. 2.5mg Compound A hydrochloride, and 1.5 mg 40-O-(2-hydroxyethyl)-rapamycin bid, the doses being adjusted as required. All patients receive corticosteroids perioperatively and continuing daily for the entire duration of the one-year study. During the one-year study period, patient visits occur on Days 0, 1,7, 14, 28 and at Months 2, 3, 6, 9 and 12. An interim analysis of efficacy and safety will be performed when all patients have completed 3 months on study, with a final analysis at 12 months post-transplantation. The renal function is evaluated by serum creatinine measurements and incidence of proteinuria within 3 and 12 months in patients. A beneficial effect is observed.

Daily dosages required in practicing the method of the present invention will vary depending upon, for example, the ALH agent employed, the host, the mode of administration, the severity of the condition to be treated, and the optionally concomitantly used immunosuppressive drug e.g. rapamycin or a derivative thereof. A preferred daily dosage range is about from 0.03 to 2.5 mg/kg per day as a single dose or in divided doses. Suitable daily dosages for patients are on the order of from e.g. 0.5 to 50 mg p.o. Suitable unit dosage forms for oral administration comprise from ca. 0.1 to 25 mg active ingredient, e.g. Compound A, e.g. in hydrochloride form, together with one or more pharmaceutically acceptable diluents or carriers therefor. As an alternative, the ALH agent may also be administered twice or three times a week, e.g. at a dosage as indicated above.

The ALH agents may be administered by any conventional route, in particular enterally, e.g. orally, for example in the form of solutions for drinking, tablets or capsules or parenterally, for example in the form of injectable solutions or suspensions. Pharmaceutical compositions comprising a compound of formula I may be manufactured in conventional manner, e.g. as described in EP-A1-627,406.

Daily dosages with respect to the co-agent used will vary depending upon, for example, the compound employed, the host, the mode of administration and the severity of the condition to be treated. A preferred daily dosage range is about from 0.25 to 25 mg of macrocyclic lactone as a single dose or in divided doses. Suitable daily dosages for patients are on the order of from e.g. 0.2 to 25 mg p.o. rapamycin or 40-O-(2-hydroxyethyl)-rapamycin, preferably 0.75 to 5 mg per day.. The co-agent b) may be administered by any conventional route, in particular enterally, e.g. orally, e.g. in the form of tablets, capsules, drink solutions, nasally, pulmonary (by inhalation) or parenterally, e.g. in the form of injectable solutions or suspensions. Suitable unit dosage forms for oral administration comprise from ca. 0.05 to 12.5 mg rapamycin or 40-O-(2-hydroxyethyl)-rapamycin, together with one or more pharmaceutically acceptable diluents or carriers therefor.

Compounds of formula I in free from or in pharmaceutically acceptable salt form are well tolerated at dosages required for use in accordance with the present invention. For example, the acute LD₅₀ is > 10 mg/kg p.o. in rats and monkeys.

## Claims

1. Use of an accelerated lymphocyte homing agent, in free form or in pharmaceutically acceptable salt form in the preparation of a pharmaceutical composition for use in reducing delayed graft function in a recipient of organ or tissue transplant.

2. Use of an accelerated lymphocyte homing agent, in free form or in pharmaceutically acceptable salt form, according to claim 1 for use in improving functional recovery of a transplanted organ or tissue in a recipient of organ or tissue transplant.

3. Use of an accelerated lymphocyte homing agent, in free form or in pharmaceutically acceptable salt form, according to claim 1 for use in improving glomerular filtration rate of a transplanted kidney in a recipient of kidney transplant.

4. Use according to any one of the preceding claims for use in combination with a co-agent which is an immunosuppressant or immunomodulatory drug.

5. Use according to claim 4 wherein the co-agent is other than a calcineurin inhibitor.

6. Use according to claim 5 wherein the co-agent is selected from rapamycin, a derivative thereof, mycophenolic acid, mycophenolate mofetil, CTLA4-lg and LEA29Y.

7. Use according to claim 5 or 6 wherein the co-agent is 40-O-(2-hydroxyethyl)-rapamycin.

8. Use according to any one of the preceding claims wherein the accelerated lymphocyte homing agent is a compound of formula I 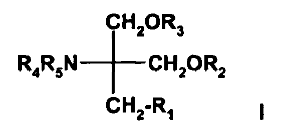 wherein
R₁ is an optionally substituted straight- or branched carbon chain having 12 to 22 carbon atoms which may be optionally interrupted by an optionally substituted phenylene, and
each of R₂, R₃, R₄ and R₅, independently, is H or lower alkyl,
in free form or in pharmaceutically acceptable salt form.

9. Use according to claim 8, wherein the compound of formula I is 2-amino-2-[2-(4-octylphenyl) ethyl]propane-1,3-diol, in free form or in pharmaceutically acceptable salt form.

10. Use according to any one of the preceding claims, wherein the graft is a non-optimal graft.

## Patentansprüche

1. Verwendung eines ALH (Accelerated Lymphocyte Homing)-Mittels in freier Form oder in pharmazeutisch annehmbarer Salzform bei der Herstellung einer pharmazeutischen Zusammensetzung zur Verwendung bei der Reduzierung einer verzögerten Transplantatfunktion bei einem Empfänger eines Organ- oder Gewebetransplantats.

2. Verwendung eines ALH-Mittels in freier Form oder in pharmazeutisch annehmbarer Salzform gemäß Anspruch 1 zur Verwendung bei der Verbesserung der funktionellen Genesung eines transplantierten Organs oder Gewebes bei einem Empfänger eines Organ- oder Gewebetransplantats.

3. Verwendung eines ALH-Mittels in freier Form oder in pharmazeutisch annehmbarer Salzform gemäß Anspruch 1 zur Verwendung bei der Verbesserung der glomerulären Filtrationsrate einer transplantierten Niere bei einem Empfänger eines Nierentransplantats.

4. Verwendung nach einem der vorstehenden Ansprüche zur Verwendung zusammen mit einem weiteren Mittel, das ein immunsuppressives oder immunmodulatorisches Arzneimittel ist.

5. Verwendung nach Anspruch 4, wobei das weitere Mittel kein Calcineurin-Inhibitor ist.

6. Verwendung nach Anspruch 5, wobei das weitere Mittel ausgewählt ist aus Rapamycin, einem Derivat davon, Mycophenolsäure, Mycophenolatmofetil, CTLA4-Ig und LEA29Y.

7. Verwendung nach Anspruch 5 oder 6, wobei das weitere Mittel für 40-O-(2-Hydroxyethyl)-rapamycin steht.

8. Verwendung nach einem der vorstehenden Ansprüche, wobei das ALH-Mittel für eine Verbindung der Formel I 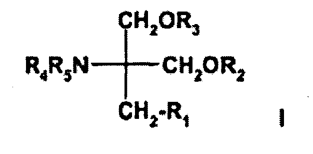 steht, wobei
R₁ für eine gegebenenfalls substituierte gerade oder verzweigte Kohlenstoffkette steht, die 12 bis 22 Kohlenstoffatome aufweist, die gegebenenfalls durch ein gegebenenfalls substituiertes Phenylen unterbrochen ist, und
jedes der R₂, R₃, R₄ und R₅ unabhängig für H oder Niederalkyl steht,
in freier Form oder in pharmazeutisch annehmbarer Salzform.

9. Verwendung nach Anspruch 8, wobei die Verbindung der Formel I für 2-Amino-2-[2-(4-octylphenyl)ethyl]propan-1,3-diol in freier Form oder in pharmazeutisch annehmbarer Salzform steht.

10. Verwendung nach einem der vorstehenden Ansprüche, wobei das Transplantat für ein nicht optimales Transplantat steht.

## Revendications

1. Utilisation d'un agent de migration lymphocytaire accélérée, sous forme libre ou sous forme de sel pharmaceutiquement acceptable dans la préparation d'une composition pharmaceutique, pour une utilisation visant à réduire la reprise différée de fonction du greffon chez un receveur d'organe ou de greffe de tissu.

2. Utilisation d'un agent de migration lymphocytaire accélérée, sous forme libre ou sous forme de sel pharmaceutiquement acceptable, selon la revendication 1, pour une utilisation visant à améliorer la récupération fonctionnelle d'un organe ou d'un tissu greffé chez un receveur d'organe ou de greffe de tissu.

3. Utilisation d'un agent de migration lymphocytaire accélérée, sous forme libre ou sous forme de sel pharmaceutiquement acceptable, selon la revendication 1, pour une utilisation visant à améliorer le débit de filtration glomérulaire d'un rein greffé chez un receveur de greffe de rein.

4. Utilisation selon l'une quelconque des revendications précédentes, pour une utilisation en combinaison avec un co-agent qui est un médicament immunodépresseur ou immunomodulateur.

5. Utilisation selon la revendication 4, dans laquelle le co-agent est autre qu'un inhibiteur de la calcineurine.

6. Utilisation selon la revendication 5, dans laquelle le co-agent est sélectionné parmi la rapamycine, un dérivé de celle-ci, l'acide mycophénolique, le mycophénolate mofétil, CTLA4-Ig et LEA29Y.

7. Utilisation selon la revendication 5 ou 6, dans laquelle le co-agent est la 40-O-(2-hydroxyéthyl)-rapamycine.

8. Utilisation selon l'une quelconque des revendications précédentes, dans laquelle l'agent de migration lymphocytaire accélérée est un composé de formule I 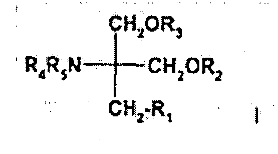 dans laquelle
R₁ représente une chaîne carbonée linéaire ou ramifiée éventuellement substituée ayant 12 à 22 atomes de carbone qui peut être éventuellement interrompue par un groupe phénylène éventuellement substitué, et chacun de R₂, R₃, R₄ et R₅, indépendamment, représente H ou un groupe alkyle inférieur,
sous forme libre ou sous forme de sel pharmaceutiquement acceptable.

9. Utilisation selon la revendication 8, dans laquelle le composé de formule I est le 2-amino-2-[2-(4-octylphényl)éthyl]propane-1,3-diol, sous forme libre ou sous forme de sel pharmaceutiquement acceptable.

10. Utilisation selon l'une quelconque des revendications précédentes, dans laquelle le greffon est un greffon non optimal.
